# EUROPÄISCHE PATENTSCHRIFT

(11) **EP 0 733 343 B1**
(45) Veröffentlichungstag und Bekanntmachung des Hinweises auf die Patenterteilung: **01.09.1999**
(21) Anmeldenummer: 96102797.6
(22) Anmeldetag: 24.02.1996
(51) Int. Cl.: A61B 17/16, B23D 71/06

(54) **Knochenraspel**
Bone rasp
Râpe à os

(30) Priorität: 24.03.1995 DE 19510735
(43) Veröffentlichungstag der Anmeldung: 25.09.1996
(73) Patentinhaber: Zeim, Ingrid, 31061 Alfeld (DE)
(72) Erfinder: Zeim, Thomas, 31073 Delligsen (DE)
(74) Vertreter: Stracke, Alexander, Dipl.-Ing.

(56) Entgegenhaltungen:
- EP-A- 0 051 577
- WO-A-93/15665
- DE-A- 3 907 256
- US-A- 507 071
- US-A- 4 598 447
- ISO 234/2 1982-05-15

## Beschreibung

Die vorliegende Erfindung betrifft eine Knochenraspel für den Einsatz bei orthopädischen Operationen zur Erzeugung eines einem einzusetzenden Implantat formmäßig entsprechenden Lagers in einem Knochen, bestehend aus einem - dem jeweiligen Einsatzzweck entsprechend geformten - Raspelkörper mit einer Vielzahl von auf seinen Außenflächen angebrachten Raspelzähnen.

Formraspeln für Knochen der vorerwähnten Art sind z.B. aus DE-A-3907256 bekannt. Im medizinischen Bereich, insbesondere bei orthopädischen Operationen, bei denen für ein einzusetzendes Implantat ein Lager in einem Knochen geraspelt werden muß, werden derartige Formraspeln eingesetzt, deren Form dem jeweiligen Einsatzzweck entsprechend vorgegeben ist.

Bei bekannten Formraspeln werden die Raspelzähne mittels eines Meißels von Hand geschlagen, der in etwa V-förmig geschliffen ist. Durch die Verwendung eines derartigen Meißels bei der Erzeugung der Formraspelzähne ergibt sich, daß die Raspelschneiden der Raspelzähne und entsprechend auch die Zahngruben eine Spitze bilden, bedingt durch die V-förmige Gestaltung des die Raspelzähne erzeugenden Meißels.

Außerdem bilden sich bei der Verwendung derart gestalteter Meißel auf dem Raspelzahn-Rücken mehr oder minder stark ausgeprägte Quetschfalten.

Die bekannte Form der Raspelzähne ist insbesondere im orthopädischen Bereich problematisch.

Einerseits ist die Reinigung derartiger Formraspeln schwierig durchzuführen, da die Zahngruben der Formraspelzähne in einer spitzen Ecke auslaufen. Die Säuberung dieser Ecken ist erfahrungsgemäß äußerst schwierig durchführbar. Dabei kommt es gerade bei Formraspeln für orthopädische Zwecke auf absolute Sauberkeit und Sterilität an.

Soweit die Raspelzähne selbst betroffen sind, ergibt sich bei den bekannten Formraspeln der Nachteil, daß diese Raspelzähne zu "aggressiv" sind, bedingt durch die spitze Form.

Durch die Quetschfalten auf den Zahnrücken ergibt sich der zusätzliche Nachteil, daß unbeabsichtigt und in unerwünschter Weise Gewebe abgetragen wird.

Man hat zwar schon versucht, die vorstehenden Nachteile zumindest zu vermindern, und zwar durch Veränderung der Winkel der V-förmigen Raspelzähne und Zahngruben und durch unterschiedliche Anstellwinkel der erzeugenden Meißel beim Schlagen der Raspelzähne. Auf diesem Wege ist zwar eine gewisse Verminderung der spitzen Raspelzahnform erreichbar, die grundsätzlichen Nachteile, die oben beschrieben sind, bleiben aber bestehen.

Der vorliegenden Erfindung liegt insoweit die Aufgabe zugrunde, eine Formraspel für Knochen der gattungsgemäßen Art zu schaffen, die sowohl hinsichtlich ihres Einsatzes im orthopädischen Bereich wie auch hinsichtlich der Reinigungsmöglichkeit nach einem Gebrauch erheblich bessere Eigenschaften aufweist.

Zur Lösung dieser Aufgabe wird eine gattungsgemäße Knochenraspel vorgeschlagen, die dadurch gekennzeichnet ist, daß die Raspelzähne mittels eines abgerundeten Meißels von Hand geschlagen erzeugt sind und die Raspelschneiden der Raspelzähne ebenso wie die Zahngruben eine gleichmäßige, vorzugsweise kreisbogenförmige Krümmung aufweisen.

Eine Knochenraspel mit derart erzeugten und entsprechend gestalteten Raspelzähnen weist eine Reihe von entscheidenden Vorteilen auf. Durch die gleichmäßige Krümmung der Raspelschneiden wird einer derartigen Formraspel die Aggressivität genommen, so daß ein Operateur erheblich gefühlvoller arbeiten kann. Weiterhin hat sich gezeigt, daß bei der Erzeugung der gekrümmten Raspelzähne Quetschfalten auf den Zahnrücken praktisch vermieden werden, so daß die durch Quetschfalten auf den Zahnrücken entstehenden Nachteile somit somit ebenfalls erheblich vermindert werden.

Ein weiterer, enorm wichtiger Vorteil der erfindungsgemäßen Knochenraspel besteht darin, daß sich die Zähne beim Arbeiten mit einer derartigen Knochenraspel weniger zusetzen, als dies bei bekannten Raspelzahn-Formen der Fall ist, so daß sich eine effektivere Arbeitsweise ergibt. Das angestrebte Ergebnis wird somit im Endeffekt schneller und schonender erreicht.

Außerdem bewirkt die erfindungsgemäße Ausführung der Raspelzähne, daß die Gefahr des Abbrechens von Zähnen beim Raspeln erheblich reduziert wird.

Die erfindungsgemäße Knochenraspel kann nach einer Benutzung problemlos und gründlichst gereinigt werden kann, da im Bereich der kritischen Zahngruben keine Ecke mehr vorhanden ist, sondern nur eine gleichmäßige, vorzugsweise kreisbogenförmige Krümmung.

Ein Ausführungsbeispiel der Erfindung ist in der beigefügten Zeichnung dargestellt und wird im folgenden näher beschrieben.

Es zeigen:
- Figur 1: eine Ansicht einer erfindungsgemäßen Knochenraspel,
- Figur 2: einen Teilschnitt nach der Linie II/II in Figur 1 in stark vergrößerter Darstellung,
- Figur 3: einen Schnitt nach der Linie III/III in Figur 2,
- Figur 4: eine perspektivische Darstellung eines Raspelzahnes der erfindungsgemäßen Knochenraspel.

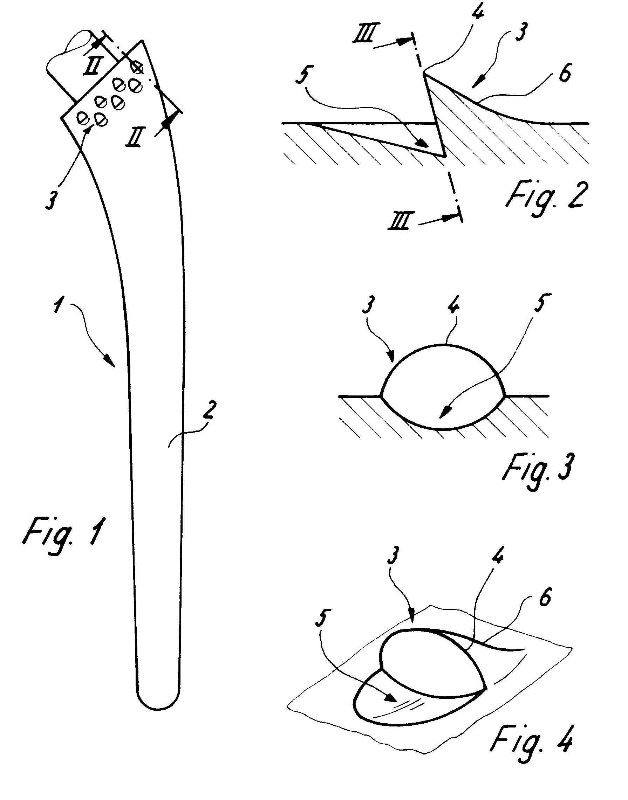

In Figur 1 ist mit dem Bezugszeichen 1 eine Knochenraspel für orthopädische Zwecke bezeichnet, die aus einem Raspelkörper 2 mit einer Vielzahl von auf seinen Außenflächen angebrachten Raspelzähnen 3 besteht.

Die Raspelzähne 3 werden von einem Meißel von Hand geschlagen und erfindungsgemäß von einem abgerundeten Meißel erzeugt, so daß die Raspelzähne 3, wie aus den Figuren 2 bis 4 sehr deutlich hervorgeht, eine Raspelschneide 4 aufweisen, die ebenso wie die Zahngruben 5 eine gleichmäßige, vorzugsweise kreisbogenförmige Krümmung aufweisen.

Durch die entsprechende runde Form der Raspelzähne 3 wird im Bereich der Zahnrücken 6 die Bildung von Quetschfalten beim Schlagen der Raspelzähne 3 vermieden.

Durch die abgerundeten Raspelzähne 3 wird ein sehr gefühlvolles Arbeiten mit der erfindungsgemäßen Knochenraspel 1 ermöglicht, da die Schnittwirkung einer derart gestalteten Knochenraspel 1 nicht so aggressiv ist, wie dies bei herkömmlichen Raspelzähnen, die in einer Spitze auslaufen, der Fall ist. Die Zahngruben 5 lassen sich besonders gut, leicht und gründlich reinigen, da diese Zahngruben 5 keine Ecken aufweisen, wie dies bei herkömmlichen Raspelzahnformen der Fall ist.

Die Zahngruben 5, die letztendlich eine spiegelbildliche Form der Raspelzähne 3 aufweisen, sind lediglich durch eine durchlaufende gekrümmte Linie begrenzt, längs derer eine exakte und vollständige Reinigung möglich ist.

Eine entsprechend konzipierte Formraspel ist somit insbesondere für orthopädische Zwecke hervorragend geeignet und erfüllt alle Ansprüche, die gerade in diesem äußerst sensiblen Einsatzbereich gestellt werden.

## Patentansprüche

1. Knochenraspel für den Einsatz bei orthopädischen Operationen zur Erzeugung eines einem einzusetzenden Implantat formmäßig entsprechenden Lagers in einem Knochen, bestehend aus einem - dem jeweiligen Einsatzzweck entsprechend geformten - Raspelkörper mit einer Vielzahl von auf seinen Außenflächen angebrachten Raspelzähnen (3), **dadurch gekennzeichnet, daß** die Raspelzähne (3) mittels eines abgerundeten Meißels von Hand geschlagen erzeugt sind und die Raspelschneiden (4) der Raspelzähne (3) ebenso wie die mittels des Meißels erzeugten Zahngruben (5) eine gleichmäßige, vorzugsweise kreisbogenförmige Krümmung aufweisen.

2. Knochenraspel nach Anspruch 1, **dadurch gekennzeichnet, daß** die Zahnrücken (6) der Raspelzähne (3) weitgehend quetschfaltenfrei sind.

## Claims

1. A bone rasp for use in orthopaedic operations for producing a mounting, corresponding in respect of shape to an implant to be inserted, in a bone, comprising a rasp body which is shaped in accordance with the respective purpose of use and having a plurality of rasp teeth (3) which are disposed on its outside surfaces characterised in that the rasp teeth (3) are produced by means of a rounded-off chisel hit by hand and the rasp cutting edges (4) of the rasp teeth (3) as well as the tooth cavities (5) produced by means of the chisel have a uniform, preferably arcuate curvature.

2. A bone rasp according to claim 1 characterised in that the backs (6) of the rasp teeth (3) are substantially crease-free.

## Revendications

1. Râpe à os destinée à être utilisée lors d'opérations orthopédiques pour former, dans un os, un logement présentant une forme correspondant à un implant à poser, la râpe comprenant un corps, dont la forme correspond à l'utilisation prévue, présentant une pluralité de dents (3) sur ses faces extérieures, caractérisée en ce que les dents (3) de la râpe sont formées, à la main, au moyen d'un outil arrondi, et en ce que les tranchants (4) des dents (3), ainsi que les creux (5) des dents formés au moyen de l'outil, présentent une courbure régulière, de préférence en arc de cercle.

2. Râpe à os selon la revendication 1, caractérisée en ce que les dos (6) des dents (3) de la râpe sont pratiquement dépourvus de plis.
